# EUROPÄISCHE PATENTANMELDUNG

(11) **EP 2 407 168 A1**
(43) Veröffentlichungstag der Anmeldung: **18.01.2012**
(21) Anmeldenummer: 11180809.3
(22) Anmeldetag: 03.05.2007
(51) Int. Cl.: A61K 31/522, A61K 31/4985, A61P 3/10, A61P 9/04

(54) **Verwendungen von DPP IV Inhibitoren**

(30) Priorität: 04.05.2006 EP 06009203
(62) Teilanmeldung aus: 07728723.3
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Dugi, Klaus, 01324 DRESDEN (DE); Himmelsbach, Frank, 88441 MITTELBIBERACH (DE); Mark, Michael, 88400 BIBERACH (DE)
(74) Vertreter: Hammann, Heinz

(57) **Zusammenfassung**

Beschrieben wird die Verwendung ausgewählter DPP IV Inhibitoren zur Behandlung physiologischer Funktionsstörungen sowie zur Reduktion des Risikos eines Eintretens solcher Funktionsstörungen in gefährdeten Patientengruppen. Zusätzlich wird die Verwendung der genannten DPP IV Inhibitoren in Kombination mit weiteren Wirkstoffen beschrieben, wodurch verbesserte Behandlungsergebnisse erzielt werden können. Diese Verwendungen lassen sich zur Herstellung entsprechender Arzneimittel nutzen.

## Beschreibung

Beschrieben wird die Verwendung ausgewählter DPP IV Inhibitoren zur Behandlung physiologischer Funktionsstörungen sowie zur Reduktion des Risikos eines Eintretens solcher Funktionsstörungen in gefährdeten Patientengruppen. Zusätzlich wird die Verwendung der genannten DPP IV Inhibitoren in Kombination mit weiteren Wirkstoffen beschrieben, wodurch verbesserte Behandlungsergebnisse erzielt werden können. Diese Verwendungen lassen sich zur Herstellung entsprechender Arzneimittel nutzen.

Das Enzym DPP-IV, auch bekannt unter der Bezeichnung CD26, ist eine Serinprotease, die bei Proteinen mit einem Prolin oder Alanin Rest am N-terminalen Ende die Abspaltung von Dipeptiden begünstigt. DPP-IV Inhibitoren beeinflussen dadurch den Plasmaspiegel bioaktiver Peptide einschließlich des Peptids GLP-1 und gelten als viel versprechende Moleküle zur Behandlung von Diabetes mellitus.

Den Autoimmunerkrankungen zugeordnet wird der Typ 1 Diabetes mellitus, der meist bei Jugendlichen unter 30 Jahren auftritt. Bei entsprechender genetischer Disposition und unter dem Einfluss verschiedener Faktoren kommt es zu einer Insulitis mit nachfolgender Zerstörung der B-Zellen, sodass der Pankreas nur mehr wenig oder kein Insulin produzieren kann.

Typ 2 Diabetes mellitus wird nicht als Autoimmunerkrankung angesehen und manifestiert sich in einem Nüchternblutzuckerwert, der 125 mg Glucose pro dl Plasma überschreitet, wobei die Bestimmung von Glucosewerten im Blut ein Standardverfahren in der medizinischen Routineanalytik darstellt. Ein Verdacht auf Prädiabetes liegt dann vor, wenn der Nüchternblutzuckerwert über dem maximalen Normalwert von 99 mg Glucose pro dl Plasma liegt aber den für Diabetes relevanten Grenzwert von 125 mg Glucose pro dl Plasma nicht überschreitet. Man spricht dann auch von pathologischer Nüchtern-Glukose (impaired fasting glucose). Ein anderes Indiz für Prädiabetes ist eine gestörte Glukose Toleranz, d.h. ein Blutzuckerwert von 140-199 mg Glucose pro dl Plasma 2 Stunden nach nüchterner Einnahme von 75 g Glucose im Rahmen eines oralen Glukose Toleranz Tests.

Wird eine oraler Glukose Toleranz Test durchgeführt, so liegt 2 Stunden nach nüchterner Einnahme von 75 g Glucose der Blutzuckerwert eines Diabetikers über 199 mg Glucose pro dl Plasma. Bei einem Glukose Toleranz Test werden der zu untersuchenden Person nach 10-12 stündigem Fasten 75 g Glucose oral verabreicht und der Blutzuckerwert wird unmittelbar vor Einnahme sowie 1 bzw. 2 Stunden nach Einnahme der Glucose ermittelt. Bei einem Gesunden liegt der Blutzuckerwert vor Einnahme zwischen 60 und 99 mg pro dl Plasma, eine Stunde nach Einnahme unter 200 mg pro dl und nach 2 Stunden unter 140 mg pro dl. Liegt der Wert nach 2 Stunden zwischen 140 und 199 mg so spricht man auch von einer gestörten Glukosetoleranz oder manchmal von einer Glukose-Intoleranz.

In der Therapieüberwachung des Diabetes mellitus hat der HbA1 c-Wert, das Produkt einer nicht-enzymatischen Glykierung der Hämoglobin B-Kette, eine überragende Bedeutung. Da seine Bildung im Wesentlichen von der Blutzuckerhöhe und der Lebenszeit der Erythrozyten abhängt, reflektiert der HbA1 c im Sinne eines "Blutzuckergedächtnisses" den mittleren Blutzuckerspiegel der vergangenen 4-12 Wochen. Diabetische Patienten, deren HbA1 c-Wert durch intensivierte Diabetestherapie dauerhaft gut eingestellt ist (d.h. < 6,5 % des gesamten Hämoglobins der Probe), sind deutlich besser vor diabetischer Mikroangiopathie geschützt. Durch verfügbare Diabetes Therapien kann beim Diabetiker eine durchschnittliche Verbesserung des HbA1 c Wertes in der Größenordnung von 1,0 - 1,5 % erreicht werden. Diese Reduktion des HbA1 C Wertes ist nicht bei allen Diabetikern ausreichend, um in den gewünschten Zielbereich von < 6,5 % und bevorzugt < 6 % HbA1 c zu kommen.

Ein besonders starkes Indiz für das Vorliegen der komplexen Stoffwechselstörung eines Prädiabetes ist, wenn eine Insulinresistenz nachgewiesen werden kann. So kann es sein, dass eine Person zur Aufrechterhaltung der Glukosehomöostase die 2-3fache Menge an endogen gebildetem Insulin benötigt als eine andere Person. Als die aussagekräftigste Methode zur Bestimmung der Insulinresistenz gilt der euglykämisch-hyperinsulinämische Clamp-Test. Das Verhältnis Insulin zu Glukose wird im Rahmen einer kombinierten Insulin-Glukose-Infusionstechnik bestimmt. Von einer Insulinresistenz spricht man, wenn die Glucoseaufnahme unterhalb der 25. Perzentile der untersuchten Hintergrundspopulation liegt (WHO Definition). Etwas weniger aufwendig als die Clamp-Untersuchung sind so genannte Minimalmodelle, bei denen während eines intravenösen Glukosetoleranz-Tests in zeitlich festgelegten Abständen Insulin- und Glukosekonzentrationen im Blut gemessen werden und hieraus die Insulinresistenz berechnet wird. Ein weiteres Bestimmungsverfahren ist das mathematische HOMA-Modell. Die Insulinresistenz wird über die Nüchtern-Plasma-Glukose und die Nüchtern-Insulin-Konzentration berechnet. Im Rahmen dieser Methode ist es nicht möglich zwischen hepatischer und peripherer Insulinresistenz zu unterscheiden. Für eine Bewertung der Insulinresistenz in der täglichen Praxis sind diese Verfahren wenig geeignet. Im klinischen Alltag werden zur Einschätzung der Insulinresistenz in der Regel andere Parameter herangezogen. Bevorzugt wird dafür z.B. die Plasma-Triglyzerid-Konzentration des Patienten herangezogen werden, da erhöhte Triglyzerid-Spiegel signifikant mit dem Vorliegen einer Insulinresistenz korrelieren.

In der Praxis geht man etwas vereinfachend davon aus, dass Personen dann Insulinresistent sind, wenn sie mindestens 2 der folgenden Eigenschaften aufweisen:
1) Übergewicht oder Fettleibigkeit
2) Bluthochdruck
3) Dyslipidaemie (veränderter Gehalt des Blutes an Gesamtlipiden)
4) mindestens einen Verwandten ersten Grades, bei dem eine gestörte Glukosetoleranz oder Typ 2 Diabetes diagnostiziert wurde.

Übergewicht bedeutet hier, dass der Body Mass Index (BMI) zwischen 25 und 30 kg/m² beträgt, wobei der BMI den Quotienten des Körpergewichts in kg und des Quadrats der Körpergröße in Metern darstellt. Bei manifester Adipositas ist der BMI 30 kg/m² oder größer.

Aus obiger Definition der Insulin Resistenz wird unmittelbar ersichtlich, dass für ihre Behandlung Blutdrucksenker dann geeignet und angezeigt sind, wenn beim Patienten u.a. die Eigenschaft Bluthochdruck festgestellt wird.

Ein ähnliches Indiz für Prädiabetes ist, wenn die Bedingungen für das Metabolische Syndrom erfüllt sind, dessen Hauptmerkmal die Insulin Resistenz ist. Gemäß den ATP IHINCEP Richtlinien (Executive Summary of the Third Report of the National Cholesterol Education Program (NCEP) im Journal of the American Medical Association 285:2486-2497, 2001) spricht man vom Metablischen Syndrom, wenn ein Patient mindestens 3 der folgenden Eigenschaften aufweist:
1) Abdominale Fettleibigkeit, definiert als Taillienumfang >40 inches oder 102 cm bei Männern und >35 inches oder 94 cm bei Frauen
2) Triglyceridwerte >150 mg/dl
3) HDL-cholesterol Werte <40 mg/dl bei Männern
4) Bluthochdruck >130/>85 mm Hg
5) Nüchternblutzucker von >110 mg/dl

Aus dieser Definition des Metabolischen Syndroms wird unmittelbar ersichtlich, dass für seine Behandlung Blutdrucksenker dann geeignet sind, wenn beim Patienten u.a. Bluthochdruck festgestellt wird.

Auch ein Blutwert für Triglyzeride von mehr als 150 mg/dl lässt das Vorliegen von Prädiabetes vermuten. Dieser Verdacht wird durch niedrige Blutwerte für HDL-Cholesterin bestärkt. Bei Frauen sind Werte unter 55 mg pro dl Plasma, bei Männern Werte unter 45 mg pro dl Plasma als zu niedrig zu werten. Die Bestimmung von Triglyzeriden und HDL-Cholesterin im Blut gehört ebenfalls zu den Standardverfahren in der medizinischen Analytik und wird z.B. in: Thomas L (Hrsg.): Labor und Diagnose", TH-Books Verlagsgesellschaft mbH, Frankfurt/Main, 2000 beschrieben. Weiter bestärkt wird ein Verdacht auf Prädiabetes, wenn die Nüchternblutzuckerwerte gleichzeitig 99 mg Glucose pro dl Plasma überschreiten.

Als Gestations-Diabetes (Schwangerschaftsdiabetes) bezeichnet man eine Form der Zuckerkrankheit, die während der Schwangerschaft entsteht und unmittelbar nach der Geburt meist wieder sistiert Diagnostiziert wird ein Gestations-Diabetes mittels eines Screening-Tests, der zwischen der 24. und 28. Schwangerschaftswoche durchgeführt wird. Dabei handelt es sich meist um den einfachen Suchtest, bei dem eine Stunde nach Verabreichung von 50 g Glukoselösung der Blutzuckerwert bestimmt wird. Liegt dieser 1 h-Wert über 140 mg/dl, so besteht der Verdacht auf Vorliegen eines Gestations-Diabetes. Die endgültige Klärung kann durch einen Standard Glukosetoleranztest mit 75 g Glukose erfolgen.

Hyperglykämie beschreibt eine Funktionsstörung bei der ein zu hoher GlukoseSpiegel im Blut gemessen wird und zwar entweder im Nüchternzustand (erhöhter Glukose Wert von 100-125 mg/dl bzw. diabetisch-hyperglykämischer Wert von >125 mg/dl verglichen mit dem normalen Wert <100 mg/dl,) oder in nicht-nüchternem Zustand (erhöhter Glukose Wert von >180 mg/dl).

Unter einem Adrenergen postprandialen Syndrom (reaktive Hypoglykämie) versteht der Mediziner eine Funktionsstörung bei der ein unverhältnismäßig hoher Insulinspiegel zu einer Absenkung des Blutzuckerspiegels (Hypoglykämie) führt verursacht durch ein Missverhältnis zwischen rasch abgebauten Kohlehydraten und einem nach einer Mahlzeit länger anhaltenden hohen Insulinspiegeln.

Als diabetischer Fuß bezeichnet man Läsionen am Fuß infolge eines Diabetes mellitus, dessen primäre Ursache eine Polyneuropathie ist, die auf eine ungenügende Stoffwechselkontrolle zurückgeht. Diagnostiziert wird ein diabetischer Fuß durch das Auftreten von typischen Läsionen (z.B. Ulzera) bei einem bestehenden Diabetes mellitus.

Als Diabetes-assoziiertes Ulkus bezeichnet man einen geschwürigen entzündlichen Defekt der Haut bei einem Patienten mit Diabetes mellitus. Diagnostiziert wird ein Diabetes-assozieertes Ulkus anhand der typischen Anamnese und körperlichen Untersuchung (z.B. Fuß-Inspektion).

Von einer diabetische Hyperlipidämie spricht man, wenn es bei einem Patienten mit Diabetes mellitus zu einer Erhöhung des Gesamtcholesterins, oder typischer bei der diabetischen Hyperlipidämie zu einer Erhöhung der Plasma-Triglyzeride mit oder ohne Erniedrigung des HDL-Cholesterins gekommen ist.

Von einer diabetischen Dyslipidämie spricht man, wenn zwar das Gesamtcholesterin nicht erhöht, aber die Verteilung auf HDL- und LDL-Cholesterin verändert ist, d.h. der Patient einen zu niedrigen HDL-Cholesterin Wert hat (z.B. <55 mg/dl für Frauen und <45 mg/dl für Männer).

Von einer Herzinsuffizienz spricht man, wenn entweder subjektive Symptome oder objektive Befunde auf eine Schwäche des Herzens hindeuten, die benötigte Auswurfleistung zu bewerkstelligen. Subjektive Symptome können z.B. Atemnot unter Belastung oder in Ruhe sein. Objektive Befunde schließen ein eine verminderte Auswurfleistung des Herzens im Ultraschall (verminderte Ejektions-Fraktion), Lungenstauung im Röntgenbild, und/oder eine verminderte Gehstrecke.

Einige ausgewählte DPP IV Inhibitoren eignen sich besonders für die Herstellung eines Arzneimittel zur therapeutischen Behandlung von Patienten, bei denen eine medizinische bzw. physiologische Funktionsstörung diagnostiziert wurde, die ausgewählt ist aus der Gruppe Prä-Diabetes, Glukose-Intoleranz (impaired glucose tolerance), pathologischer Nüchtern-Glukose (impaired fasting glucose), diabetischer Fuß, Diabetes-assoziierter Ulkus, diabetische Hyperlipidämie, diabetische Dyslipidämie, neu diagnostizierter Typ 1 Diabetes (zur Erhaltung einer restlichen Insulin-Sekretion der Bauchspeicheldrüse), Gestations-Diabetes (Schwangerschaftsdiabetes), Hyperglykämie, Adrenerges postprandiales Syndrom (reaktive Hypoglykämie) oder Herzinsuffizienz.

Diese Arzneimittel können auch dazu verwendet werden, das Risiko zu mindern, dass es trotz Therapie zu einer verschlechterten Glukose-Stoffwechsellage, einem erhöhten HbA1c-Wert, einem verschlechterten Nüchternglukose Wert, einem manifesten Typ 2 Diabetes, einem diabetischen Fuß, einem Diabetes-assoziierten Ulkus, einer diabetischen Hyperlipidämie oder einer diabetischen Dyslipidämie kommt, dass trotz Therapie eine Insulin-Behandlung notwendig wird, oder dass makrovaskuläre Komplikationen auftreten.

Beispiele solcher makrovaskulärer Komplikationen sind Myokardinfarkt, akutes Koronar-Syndrom, instabile Angina Pectoris, stabile Angina pectoris, hämorrhagischer oder ischämischer Schlaganfall, periphere arterielle Verschlusskrankheit, Kardiomyopathie, Linksherzinsuffizienz, Rechtsherzinsuffizienz, globale Herzinsuffizienz, Herzrhythmusstörungen und vaskuläre Restenose. Diese makrovaskuläre Komplikationen sind dem Fachmann bekannt und in den gängigen Lehrbüchern ausführlich beschrieben.

Darüber hinaus sind die Substanzen geeignet, nach erfolgter Transplantation von Langerhansschen Inseln oder Beta-Zellen, die Vitalität und Sekretionskapazität der Zellen zu stärken und damit eine n günstigen Verlauf nach Transplantation zu gewährleisten. Daneben können die Substanzen auch während der Isolierungs- und Transplantationsphase von Langerhansschen Inseln oder Beta Zelen eingesetzt werden, indem dem üblichen Isolations- oder Aufbewahrungsmedium die genannten Substanzen in geeigneter Konzentration von 1 nmol/l bis 1 µmol/l zugesetzt werden, vorzugsweise in einer Konzentration von 1 nmol/l bis 100 nmol/l. Damit wird eine Verbesserung der Qualität des zu transplantierenden Materials erreicht. Insbesondere in Kombination mit zugegebenen Mengen von GLP-1 (Glucagon like peptide 1), vorzugsweise in einer Konzentration von 1-100 nmol/l wird eine Qualitätsverbesserung erreicht. Entsprechende Isolations- oder Aufbewahrungsmedien sowie entsprechende Verfahren zur Stärkung der Vitalität und Sekretionskapazität von Langerhansschen Inseln oder Beta Zellen durch Zugabe von DPP IV Inhibitoren zu den verwendeten Medien sind ein weiterer Gegenstand der Erfindung.

Schließlich eignen sich die genannten In hibitoren zur Behandlung verschiedener Formen von Arthritis, vor allem aber rheumatoider Arthrithis.

Gemäß der vorliegenden Erfindung ausgewählte DPP IV Inhibitoren lassen sich beschreiben durch die Formel (I) 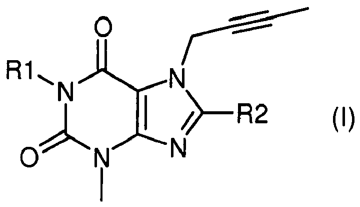 oder Formel (II) 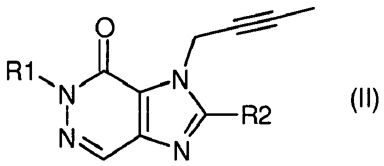 worin **R1** ([1,5]Naphthyridin-2-yl)methyl, (Chinazolin-2-yl)methyl, (Chinoxalin-6-yl)methyl, (4-Methyl-chinazolin-2-yl)methyl, 2-Cyano-benzyl, (3-Cyano-chinolin-2-yl)methyl, (3-Cyano-pyridin-2-yl)methyl, (4-Methyl-pyrimidin-2-yl)methyl, oder (4,6-Dimethyl-pyrimidin-2-yl)methyl bedeutet und **R2** 3-(*R*)-amino-piperidin-1-yl, (2-amino-2-methyl-propyl)-methylamino oder (2-(S)-amino-propyl)-methylamino.

Besonders bevorzugte DPP IV Inhibitoren sind die folgenden Verbindungen und deren therapeutisch wirksamen Salze:
- 1-[(4-Methyl-chinazolin-2-yl)methyl]-3-methyl-7-(2-butin-1-yl)-8-(3-(*R*)-amino-piperidin-1-yl)-xanthin (siehe WO 2004/018468, Beispiel 2(142): 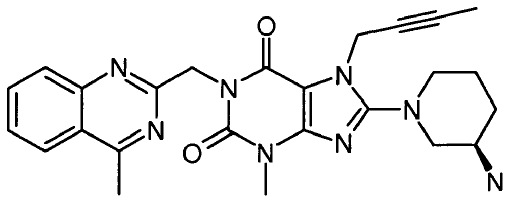
- 1-[([1,5]Naphthyridin-2-yl)methyl]-3-methyl-7-(2-butin-1-yl)-8-((*R*)-3-amino-piperidin-1-yl)-xanthin (siehe WO 2004/018468, Beispiel 2(252)): 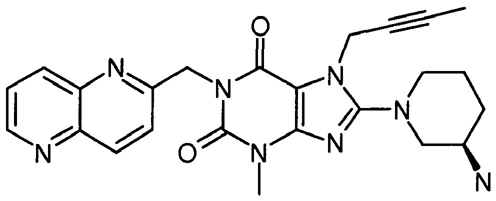
- 1-[(Chinazolin-2-yl)methyl]-3-methyl-7-(2-butin-1-yl)-8-((*R*)-3-amino-piperidin-1-yl)-xanthin (siehe WO 2004/018468, Beispiel 2(80)): 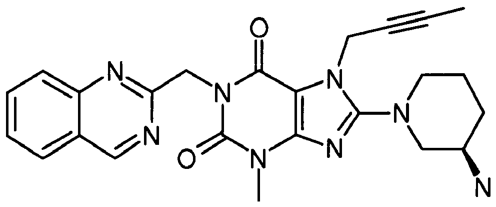
- 2-((R)-3-Amino-piperidin-1-yl)-3-(but-2-inyl)-5-(4-methyl-chinazolin-2-ylmethyl)-3,5-dihydro-imidazo[4,5-d]pyridazin-4-on (siehe WO 2004/050658, Beispiel 136): 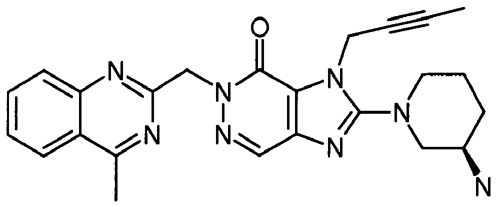
- 1-[(4-Methyl-chinazolin-2-yl)methyl]-3-methyl-7-(2-butin-1-yl)-8-[(2-amino-2-methyl-propyl)-methylamino]-xanthin (siehe WO 2006/029769, Beispiel 2(1)): 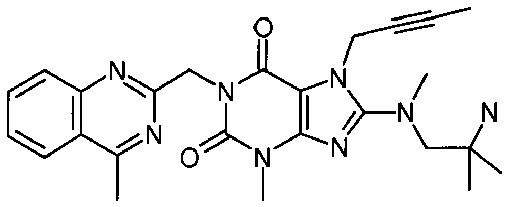
- 1-[(3-Cyano-chinolin-2-yl)methyl]-3-methyl-7-(2-butin-1-yl)-8-((*R*)-3-amino-piperidin-1-yl)-xanthin (siehe WO 2005/085246, Beispiel 1 (30)): 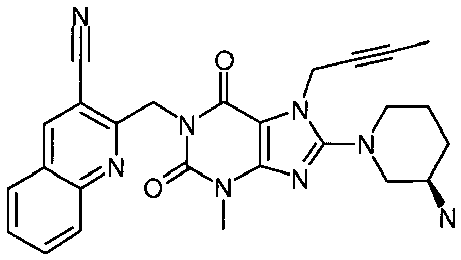
- 1-(2-Cyano-benzyl)-3-methyl-7-(2-butin-1-yl)-8-((*R*)-3-amino-piperidin-1-yl)-xanthin (siehe WO 2005/085246, Beispiel 1 (39)): 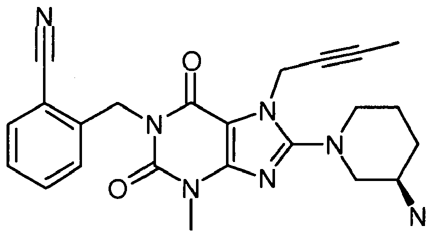
- 1-[(4-Methyl-chinazolin-2-yl)methyl]-3-methyl-7-(2-butin-1-yl)-8-[(*S*)-(2-amino-propyl)-methylamino]-xanthin (siehe WO 2006/029769, Beispiel 2(4)): 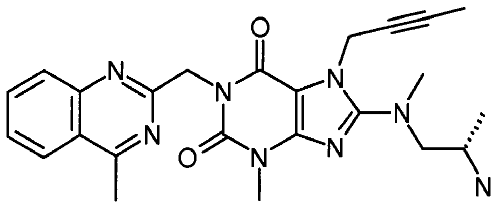
- 1-[(3-Cyano-pyridin-2-yl)methyl]-3-methyl-7-(2-butin-1-yl)-8-((*R*)-3-amino-piperidin-1-yl)-xanthin (siehe WO 2005/085246, Beispiel 1 (52)): 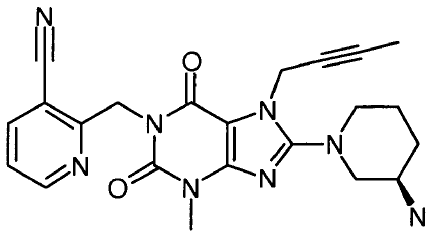
- 1-[(4-Methyl-pyrimidin-2-yl)methyl]-3-methyl-7-(2-butin-1-yl)-8-((*R*)-3-amino-piperidin-1-yl)-xanthin (siehe WO 2005/085246, Beispiel 1(81)): 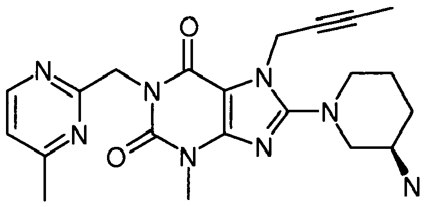
- 1-[(4,6-Dimethyl-pyrimidin-2-yl)methyl]-3-methyl-7-(2-butin-1-yl)-8-((*R*)-3-amino-piperidin-1-yl)-xanthin (siehe WO 2005/085246, Beispiel 1 (82)): 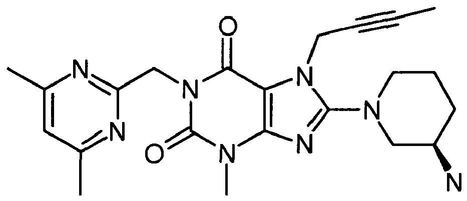
- 1-[(Chinoxalin-6-yl)methyl]-3-methyl-7-(2-butin-1-yl)-8-((*R*)-3-amino-piperidin-1-yl)-xanthin (siehe WO 2005/085246, Beispiel 1 (83)):

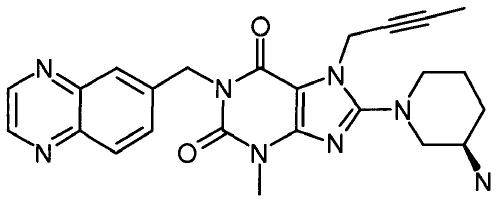

Diese DPP IV Inhibitoren heben sich von strukturell vergleichbaren DPP IV Inhibitoren ab, da sie eine herausragende Wirkstärke und Wirkdauer mit günstigen pharmakologische Eigenschaften, Rezeptorselektivität und günstigem Nebenwirkungsprofil verbinden oder in Kombination mit anderen pharmazeutischen Wirkstoffen unerwartete Vorteile oder Verbesserungen in der Therapie bewirken. Ihre Herstellung wird in den angegebenen Dokumenten offenbart.

Da unterschiedliche metabolische Funktionsstörungen oft gleichzeitig auftreten, ist es nicht selten angezeigt, mehrere unterschiedliche Wirkprinzipien miteinander zu kombinieren. So können in Abhängigkeit von den diagnostizierten Funktionsstörungen verbesserte Behandlungsergebnisse erzielt werden, wenn ein DPP IV Inhibitor kombiniert wird mit einem Wirkstoff ausgewählt aus der Gruppe anderer Antidiabetika, vor allem Wirkstoffen, die den Blutzuckerspiegel oder den Lipidspiegel im Blut senken, den HDL-Spiegel im Blut erhöhen, den Blutdruck senken oder die bei der Behandlung von Atherosklerose oder Fettleibigkeit angezeigt sind.

Die erforderliche Dosierung der DPP IV Inhibitoren beträgt bei intravenöser Gabe 0,1 mg bis 10 mg, vorzugsweise 0,25 mg bis 5 mg, und bei oraler Gabe 0.5 mg bis 100 mg, vorzugsweise 2,5 mg bis 50 mg, jeweils 1 bis 4 x täglich. Hierzu lassen sich die Verbindungen, gegebenenfalls in Kombination mit einer anderen Wirksubstanz, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die erfindungsgemäßen DPP IV Inhibitoren werden also vom Fachmann unter Verwendung zugelassener Formulierungshilfstoffe nach Verfahren hergestellt, wie sie im Stand der Technik beschrieben sind. Beispiele für solche Hilfsstoffe sind Verdünnungsmittel, Bindemittel, Träger, Füllstoffe, Gleitmittel, Verlaufmittel, Kristallisationsverzögerer, Zerfallmittel, Lösungsvermittler, farbgebende Mittel, pHregulierende Mittel, Tenside und Emulgatoren.

Geeignete Verdünnungsmittel sind zum Beispiel Zellulosepulver, Calciumhydrogenphosphat, Erythrit, (niedrig substituierte) Hydroxypropylcellulose, Mannit, Pregelatinierte Stärke oder Xylit.

Geeignete Bindemittel sind zum Beispiel Copolymere von Vinylpyrrolidon mit anderen Vinylderivaten (Copovidon), Hydroxypropylmethylcellulose (HPMC), Hydroxypropylcellulose (HPC) Polyvinylpyrrolidon (Povidon), Pregelatinierte Stärke, oder niedrig substituierte Hydroxypropylcellulose.

Geeignete Gleitmittel sind zum Beispiel Talk, Polythylenglycol, Calciumbehenat, Calciumstearat, hydriertes Rizinusöl oder Magnesiumstearat.

Geeignete Zerfallmittel sind zum Beispiel Maisstärke oder Crospovidone.

Geeignete Verfahren zur Herstellung pharmazeutischer Formulierungen der erfindungsgemässen DPP IV Inhibitoren sind
- Direkttablettierung des Wirkstoffs in Pulvermischungen mit geeigneten Tablettierhilfsstoffen;
- Granulierung mit geeigneten Hilfsstoffen und nachfolgende Mischung mit geeigneten Hilfsstoffen und anschließende Tablettierung sowie Filmcoating; oder
- Abfüllung von Pulvermischungen oder Granulaten in Kapseln.

Geeignete Granulierungsverfahren sind
- Nassgranulierung im Intensivmischer mit anschließender Wirbelschichttrocknung;
- Eintopfgranulierung;
- Wirbelschichtgranulierung; oder
- Trockengranulierung (z.B. durch Walzenkompaktierung) mit geeigneten Hilfsstoffen und anschließender Tablettierung oder Abfüllung in Kapseln.

Die genannten DPP IV Inhibitoren können auch in Kombination mit weiteren Wirkstoffen verwendet werden, wodurch sich verbesserte Behandlungsergebnisse erzielen lassen. Eine solche Kombinationsbehandlung kann als freie Kombination der Wirkstoffe oder in Form einer fixierten Kombination zum Beispiel in einer Tablette oder Kapsel erfolgen. Dafür notwendige pharmazeutische Formulierungen des Kombinationspartners sind entweder als Arzneimittel kommerziell erhältlich oder können vom Fachmann nach gängigen Verfahren formuliert werden. Die kommerziell als Arzneimittel erhältlichen Wirkstoffe sind im Stand der Technik mehrfach beschrieben, zum Beispiel im jährlich erscheinenden Arzneimittelverzeichnis "Rote Liste®" des Bundesverbandes der pharmazeutischen Industrie oder i n der jährlich aktualisierten Zusammenstellung der Herstellerinformationen zu verschreibungspflichtigen Arzneimitteln genannt "Physicians' Desk Reference".

Beispiele für antidiabetische Kombinationspartner sind Metformin; Sulfonylharnstoffe wie Glibenclamide, Tolbutamide, Glimepiride, Glipizid, Gliquidon, Glibornurid und Gliclazid; Nateglinide; Repaglinide; Thiazolidindione wie Rosiglitazone und Pioglitazone; PPAR gamma Modulatoren wie Metaglidasen; PPAR-gamma Agonisten wie GI 262570; PPAR-gamma Antagonisten; PPAR-gamma/alpha Modulatoren wie tesaglitazar, muraglitazar und KRP297; PPAR-gamma/alpha/delta Modulatoren; AMPK-Aktivatoren wie AICAR; Acetyl-CoA Carboxylase (ACC1 und ACC2) Inhibitoren; Diacylglycerol-acetyltransferase (DGAT) Inhibitoren; pankreatische beta Zell GCRP Agonisten wie SMT3-Rezeptor-Agonisten und GPR119; 11 β-HSD-Inhibitoren; FGF19 Agonisten oder Analoge; Alpha-Glucosidase Blocker wie Acarbose, Voglibose und Miglitol; Alpha2-Antagonisten; Insulin and Insulin Analoge wie Humaninsulin, Insulinlispro, Insulinglusilin, r-DNS-Insulinaspart, NPH Insulin, Insulindetemir, Insulin Zinc Suspension und Insulinglargin; Gastric Inhibitory Peptide (GIP); Pramlintide; Amylin oder GLP-1 und GLP-1 Analoge wie Exendin-4; SGLT2-Inhibitoren wie KGT-1251; Inhibitoren der Proteintyrosinphosphatase; Inhibitoren der Glucose-6-phosphatase; Fructose-1,6-bisphosphatase Modulatoren; Glycogenphosphorylase Modulatoren; Glucagon Rezeptor Antagonisten; Phosphoenolpyruvatecarboxykinase (PEPCK) Inhibitoren; Pyruvatdehydrogenasekinase (PDK) Inhibitoren; Inhibitoren von Tyrosin-Kinasen (50 mg bis 600 mg) wie der PDGF-Rezeptor-Kinase (vgl. EP-A-564409, WO 98/35958, US 5093330, WO 2004/005281, und WO 2006/041976); Glucokinase/Regulatory Protein Modulatoren incl. G lucokinase Aktivatoren; Glycogensynthasekinase Inhibitoren; Inhibitoren der SH2-domain-containing inositol 5-phosphatase type 2 (SHIP2) ; IKK Inhibitoren wie hochdosiertes Salicylat ; JNK1 Inhibitoren ; Proteinkinase C-theta Inhibitoren; Beta 3 Agonisten wie Ritobegron, YM 178, Solabegron, Talibegron, N-5984, GRC-1087, Rafabegron, FMP825; Aldosereduktase Inhibitoren wie AS 3201, Zenarestat, Fidarestat, Epalrestat, Ranirestat, NZ-314, CP-744809, und CT-112; SGLT-1 bzw. SGLT-2 Inhibitoren; KV 1.3 Kanal Inhibitoren; GPR40 Modulatoren; SCD-1 Inhibitoren; CCR-2 Antagonisten; und andere DPP IV Inhibitoren.

Beispiele für 11ß-HSD1-Inhibitoren werden beschrieben in WO 2007/013929, WO 2007/007688, WO 2007/003521, WO 2006/138508, WO 2006/135795, WO 2006/135667, WO 2006/134481, WO 2006/134467, WO 2006/132436, WO 2006/132197, WO 2006/113261, WO 2006/106423, WO 2006/106052, WO 2006/105127, WO 2006/104280, WO 2006/100502, WO 2006/097337, WO 2006/095822, WO 2006/094633, WO 2006/080533, WO 2006/074330, WO 2006/074244, WO 2006/068992, WO 2006/068991, WO 2006/068199, WO 2006/066109, WO 2006/055752, WO 2006/053024, WO 2006/051662, WO 2006/050908, WO 2006/049952, WO 2006/048750, WO 2006/048331, WO 2006/048330, WO 2006/040329, WO 2006/037501, WO 2006/030805, WO 2006/030804, WO 2006/017542, WO 2006/024628, WO 2006/024627, WO 2006/020598, WO 2006/010546, WO 2006/002349, WO 2006/002350, WO 2006/012173, WO 2006/012227, WO 2006/012226, WO 2006/000371, WO 2005/118538, WO 2005/116002, WO 2005/110992, WO 2005/110980, WO 2005/108359, WO 2005/108361, WO 2005/108360, WO 2005/108368, WO 2005/103023, WO 2005/097764, WO 2005/097759, WO 2005/095350, WO 2005/075471, WO 2005/063247, WO 2005/060963, WO 2005/047250, WO 2005/046685, WO 2005/044192, WO 2005/042513, WO 2005/016877, WO 2004/113310, WO 2004/106294, WO 2004/103980, WO 2004/089896, WO 2004/089380, WO 2004/089471, WO 2004/089470, WO 2004/089367, WO 2005/073200, WO 2004/065351, WO 2004/058741, WO 2004/056745, WO 2004/056744, WO 2004/041264, WO 2004/037251, WO 2004/033427, WO 2004/011410, WO 2003/104208, WO 2003/104207, WO 2003/065983, WO 2003/059267, WO 2003/044009, WO 2003/044000, WO 2003/043999, WO 2002/076435, WO 2001/090094, WO 2001/090093, WO 2001/090092, WO 2001/090091, WO 2001/090090, US 2007/049632, US 2006/148871, US 2006/025445, US 2006/004049, US 2005/277647, US 2005/261302, US 2005/245534, US 2005/245532, US 2005/245533 und JP 2005/170939. Ein beispielhafter Vertreter für einen 11ß-HSD1-Inhibitor ist die Verbindung: 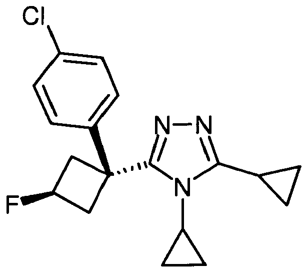 sowie dessen Salze.

Beispiele für Glycogenphosphorylase Modulatoren werden beschrieben in WO 2006/126695, WO 2006/082401, WO 2006/082400, WO 2006/059165, WO 2006/059164, WO 2006/059163, WO 2006/056815, WO 2006/055463, WO 2006/055462, WO 2006/055435, WO 2006/053274, WO 2006/052722, WO 2005/085245, WO 2005/085194, WO 2005/073231, WO 2005/073230, WO 2005/073229, WO 2005/067932, WO 2005/020987, WO 2005/020986, WO 2005/020985, WO 2005/019172, WO 2005/018637, WO 2005/013981, WO 2005/013975, WO 2005/012244, WO 2004/113345, WO 2004/104001, WO 2004/096768, WO 2004/092158, WO 2004/078743, WO 2004/072060, WO 2004/065356, WO 2004/041780, WO 2004/037233, WO 2004/033416, WO 2004/007455, WO 2004/007437, WO 2003/104188, WO 2003/091213, WO 2003/084923, WO 2003/084922, WO 2003/074532, WO 2003/074531, WO 2003/074517, WO 2003/074513, WO 2003/074485, WO 2003/074484, WO 2003/072570, WO 2003/059910, WO 2003/037864, WO 2002/096864, WO 2002/020530, WO 2001/094300, WO 2000/123347, WO 1996/39384, WO 1996/39385, EP 1391460, EP 1136071, EP 1125580, EP 1088824, EP 0978279, JP 2004196702, US 2004/002495, US 2003/195243, und US 5998463.

Beispiele für Glucokinase-Aktivatoren werden beschrieben in WO 2007/017649, WO 2007/007910, WO 2007/007886, WO 2007/007042, WO 2007/007041, WO 2007/007040, WO 2007/006814, WO 2007/006761, WO 2007/006760, WO 2006/125972, WO 2006/125958, WO 2006/112549, WO 2006/059163, WO 2006/058923, WO 2006/049304, WO 2006/040529, WO 2006/040528, WO 2006/016194, WO 2006/016178, WO 2006/016174, WO 2005/121110, WO 2005/103021, WO 2005/095418, WO 2005/095417, WO 2005/090332, WO 2005/080360, WO 2005/080359, WO 2005/066145, WO 2005/063738, WO 2005/056530, WO 2005/054233, WO 2005/054200, WO 2005/049019, WO 2005/046139, WO 2005/045614, WO 2005/044801, WO 2004/081001, WO 2004/076420, WO 2004/072066, WO 2004/072031, WO 2004/063194, WO 2004/063179, WO 2004/052869, WO 2004/050645, WO 2004/031179, WO 2004/002481, WO 2003/095438, WO 2003/080585, WO 2003/055482, WO 2003/047626, WO 2003/015774, WO 2003/000267, WO 2003/000262, WO 2002/048106, WO 2002/046173, WO 2002/014312, WO 2002/008209, WO 2001/085707, WO 2001/085706, WO 2001/083478, WO 2001/083465, WO 2001/044216, und WO 2000/058293

Beispielhafte Vertreter für Glucokinase-Aktivatoren sind die Verbindungen 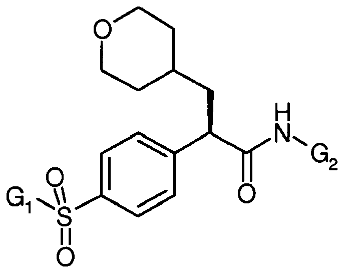 worin G₁ Cyclopropyl oder Cyclobutyl bedeutet und G₂ 5-Fluor-thiazol-2-yl, 1-Methyl-1*H*-pyrazol-3-yl, oder Pyrazin-2-yl; und 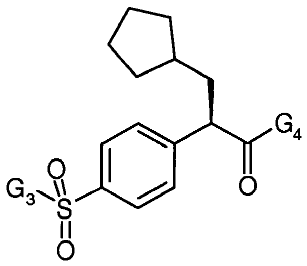 worin G₃ Methyl oder Ethyl bedeutet und G₄ Thiazol-2-yl, 4-Methyl-thiazol-2-yl, 5-Methyl-thiazol-2-yl, oder Pyrazin-2-yl sowie deren Salze.

Beispiele für SGLT1 bzw. SGLT2-Inhibitoren werden beschrieben in WO 2006/108842, WO 2006/087997, WO 2006/080577, WO 2006/080421, WO 2006/073197, WO 2006/064033, WO 2006/062224, WO 2006/054629, WO 2006/037537, WO 2006/035796, WO 2006/018150, WO 2006/008038, WO 2006/002912, WO 2006/010557, WO 2006/011502, WO 2006/011469, WO 2005/121161, WO 2005/012326, WO 2005/095429, WO 2005/095372, WO 2005/095373, WO 2005/092877, WO 2005/085267, WO 2005/085265, WO 2005/085237, WO 2005/063785, WO 2005/021566, WO 2005/012243, WO 2005/012242, WO 2005/012326, WO 2005/012318, WO 2005/011592, WO 2004/113359, WO 2004/099230, WO 2004/089967, WO 2004/089966, WO 2004/087727, WO 2004/080990, WO 2004/058790, WO 2004/052903, WO 2004/052902, WO 2004/019958, WO 2004/018491, WO 2004/014932, WO 2004/014931, WO 2004/013118, WO 2003/099836, WO 2003/080635, WO 2003/020737, WO 2003/011880, WO 2003/000712, WO 2002/098893, WO 2002/088157, WO 2002/083066, WO 2002/068440, WO 2002/068439, WO 2002/064606, WO 2002/053573, WO 2002/044192, WO 2002/036602, WO 2002/028872, WO 2001/074835, WO 2001/074834, WO 2001/068660, WO 2001/027128, WO 2001/016147, J P 2005247834, JP 2004359630, JP 2004196788, JP 2003012686, und US 2006/063722.

Beispielhafte Vertreter für SGLT1 bzw. SGLT2-Inhibitoren sind folgende Verbindungen und ihre Salze bzw. ihre Komplexe mit natürlichen Aminosäuren 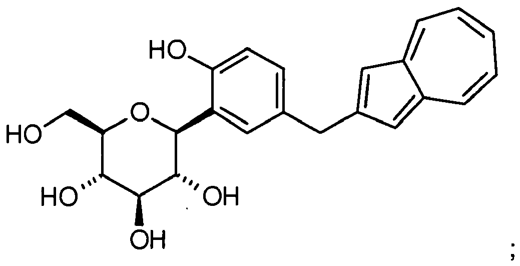 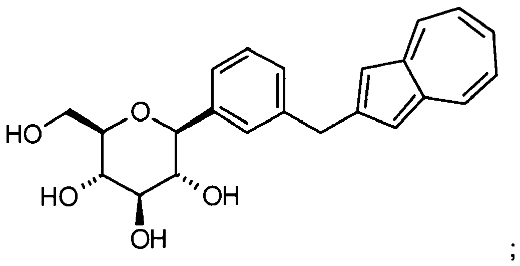 sowie 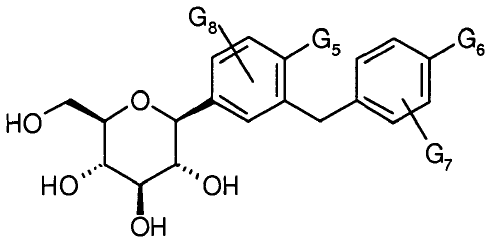 worin G₅ und G₈ unabhängig voneinander Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy oder Cyclohexyloxy bedeute n; und
G₆ Fluor, Chlor, Methyl, Ethyl, Methoxy, Ethoxy, Difuormethoxy, Trifluormethoxy, Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyl, Cyclobutyl, Cyclopentyl, Cyclohexyl, Cyclopropyloxy, Cyclobutyloxy, Cyclopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy; und G₇ Wasserstoff oder Fluor bedeutet; 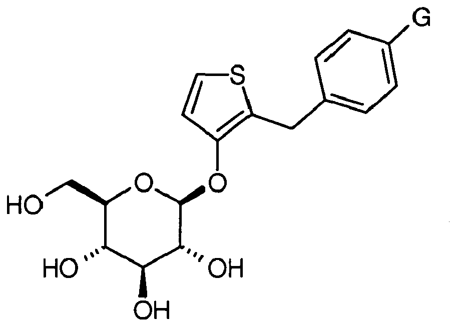 worin G Fluor, Chlor, Methyl, Ethyl, Ethinyl, Methoxy, Ethoxy, Difluormethoxy, Trifluormethoxy, Cyclobutyloxy, Cyclopentyloxy, 3-Tetrahydrofuranyloxy, oder 4-Tetrahydropyranyloxy bedeutet; 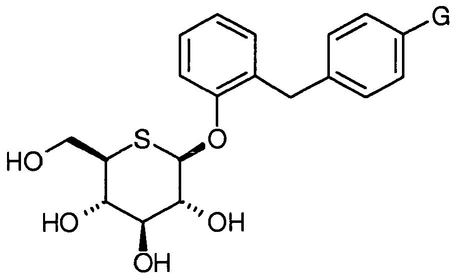 worin G Fluor, Chlor, Methyl, Ethyl, Ethinyl, Methoxy, Ethoxy, Difluormethoxy, Trifluormethoxy, Cyclobutyloxy, Cyclopentyloxy, 3-Tetrahydrofuranyloxy, oder 4-Tetrahydropyranyloxy bedeutet; 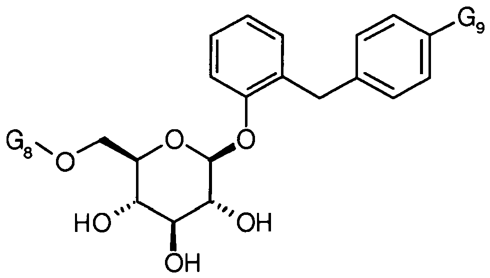 worin G₈ Wasserstoff, Methoxycarbonyl, oder Ethoxycarbonyl bedeutet und G₉ Fluor, Chlor, Methyl, Ethyl, Ethinyl, Methoxy, Ethoxy, Difluormethoxy, Trifluormethoxy, Cyclobutyloxy, Cyclopentyloxy, 3-Tetrahydrofuranyloxy, oder 4-Tetrahydropyranyloxy bedeutet sowie 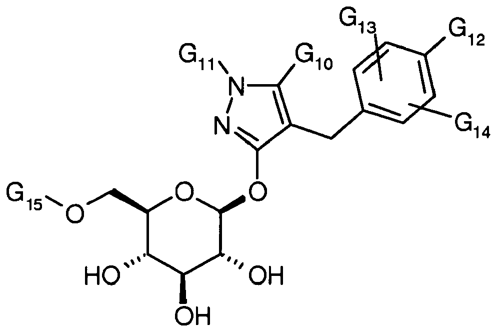 worin bedeuten:
G₁₀ C₁₋₃-Alkyl oder Perfluor-C₁₋₃-alkyl;
G₁₁ Wasserstoff, C₁₋₃-Alkyl oder Perfluor-C₁₋₃-alkyl;
G₁₂ Fluor, Chlor, Brom, C₁₋₆-Alkyl, C₁₋₆-Alkyl substituiert mit 1 bis 3 Fluoratomen, C₁₋₆-Alkoxy, C₁₋₆-Alkoxy substituiert mit 1 bis 3 Fluoratomen, C₁₋₆-Alkylthio, C₂₋₆-Alkenyl, C₂₋₆-Alkinyl, Perfluor-C₁₋₃-alkyl, Cyclobutyloxy, Cyclopentyloxy, Cyclohexyloxy, Tetrahydrofuranyloxy, oder 4-Tetrahydropyranyloxy; und
G₁₃ und G₁₄ unabhängig voneinander Wasserstoff, Fluor, Chlor, Brom, C₁₋₆-Alkyl, C₁₋₆-Alkyl substituiert mit 1 bis 3 Fluoratomen, C₁₋₆-Alkoxy, C₁₋₆-Alkoxy substituiert mit 1 bis 3 Fluoratomen, C₁₋₆-Alkylthio, C₂₋₆-Alkenyl, C₂₋₆-Alkinyl, Perfluor-C₁₋₃-alkyl; und G₁₅ Wasserstoff, C₂₋₂₀-Alkanoyl, C₁₋₆-Alkoxycarbonyl oder Benzoyl.

Ein besonders bevorzugtes Beispiel für einen antidiabetischen Kombinationspartner ist Metformin in Dosierungen von etwa 100 mg bis 500 mg oder 200 mg bis 850 mg (1-3 x täglich), oder etwa 300 mg bis 1000 mg 1-2 x täglich, oder retardiertes Metformin in Dosierungen von etwa 100 mg bis 1000 mg oder bevorzugt 500 mg bis 1000 mg 1-2 x täglich oder etwa 500 mg bis 2000 mg 1 x täglich. Ein weiteres besonders bevorzugtes Beispiel ist Pioglitazone in der Dosierung von etwa 1-10 mg, 15 mg, 30 mg, oder 45 mg 1 x täglich. Ein weiteres besonders bevorzugtes Beispiel ist Miglitol in der Dosierung von etwa 10 mg bis 50 mg oder bis zu 100 mg 1-3 x täglich.

Beispiele für Kombinationspartner, die den Lipidspiegel im Blut senken sind HMG-CoA-Reductase Hemmer wie Simvastatin, Atorvastatin, Lovastatin, Fluvastatin, Pravastatin und Rosuvastatin; Fibrate wie Bezafibrat, Fenofibrat, Clofibrat, Gemfibrozil, Etofibrat und Etofyllinclofibrat; Nicotinsäure und deren Derivate wie Acipimox; PPAR-alpha Agonisten; PPAR-delta Agonisten; Inhibitoren der Acylcoenzyme A:cholesterolacyltransferase (ACAT; EC 2.3.1.26) wie Avasimibe; Cholesterol Resorptionsinhibitoren wie Ezetimib; an Gallensäure bindende Substanzen wie Colestyramin, Colestipol und Colesevelam; Hemmer des Gallensäuretransportes; HDL modulierende Wirkstoffe wie D4F, reverse D4F, LXR modulierende Wirkstoffe und FXR modulierende Wirkstoffe; CETP Inhibitoren wie Torcetrapib, JTT-705 oder Verbindung 12 aus WO 2007/005572; LDL Rezeptor Modulatoren; und ApoB100 antisense RNA. Ein besonders bevorzugtes Beispiel ist Atorvastatin in der Dosierung von etwa 1 mg bis 40 mg oder 10 mg bis 80 mg einmal täglich.

Beispiele für Kombinationspartner, die den Blutdruck senken sind Beta-Blocker wie Atenolol, Bisoprolol, Celiprolol, Metoprolol und Carvedilol;Diuretika wie Hydrochlorothiazide, Chlortalidon, Xipamid, Furosemid, Piretanid, Torasemid, Spironolacton, Eplerenon, Amilorid und Triamteren; Kalzium Kanal Blocker wie Amlodipin, Nifedipin, Nitrendipin, Nisoldipin, Nicardipin, Felodipin, Lacidipin, Lercanipidin, Manidipin, Isradipin, Nilvadipin, Verapamil, Gallopamil und Diltiazem; ACE Hemmer wie Ramipril, Lisinopril, Cilazapril, Quinapril, Captopril, Enalapril, Benazepril, Perindopril, Fosinopril und Trandolapril; sowie Angiotensin II Rezeptor Blocker (ARBs) wie Telmisartan, Candesartan, Valsartan, Losartan, Irbesartan, Olmesartan und Eprosartan. Besonders bevorzugte Beispiele sind Metoprolol in der Dosierung von 50 mg bis 200 mg täglich, Amlodipin in der Dosierung 2,5 mg bis 10 mg täglich, Ramipril in der Dosierung von 2,5 mg bis 15 mg täglich, Valsartan in der Dosierung von 80 bis 160 mg täglich, und Telmisartan in der Dosierung 20 mg bis 320 mg oder 40 mg bis 160 mg täglich.

Beispiele für Kombinationspartner, die den HDL-Spiegel im Blut erhöhen, sind Cholesteryl Ester Transfer Protein (CETP) Inhibitoren; Inhibitoren Endothelialer Lipase; Regulatoren von ABC1; LXRalpha Antagonisten; LXRbeta Agonisten; PPAR-delta Agonisten; LXRalpha/beta Regulatoren, und Substanzen, welche die Expression und/oder Plasmakonzentration von Apolipoprotein A-I steigern.

Beispiele für Kombinationspartner zur Behandlung der Fettleibigkeit sind Sibutramine; Tetrahydrolipstatin (Orlistat); Alizyme; Dexfenfluramine; Axokine; Cannabinoid Rezeptor 1 Antagonisten wie der CB1 Antagonist rimonobant; MCH-1 Rezeptor Antagonisten; MC4 Rezeptor Agonisten; NPY5 sowie NPY2 Antagonisten; Beta3-AR Agonisten wie SB-418790 und AD-9677; 5HT2c Rezeptor Agonisten wie APD 356; Myostatin Inhibitoren; Acrp30 und Adiponectin; Steroyl CoA Desaturase (SCD1) Inhibitoren; Fettsäuresynthase (FAS) Inhibitoren; CCK Rezeptor Agonisten; Ghrelin Rezeptor Modulatoren; Pyy 3-36; Orexi n Rezeptor Antagonisten; und Tesofensine.

Beispiele für Kombinationspartner zur Behandlung der Atherosklerose sind Phospholipase A2 Inhibitoren; Inhibitoren von Tyrosin-Kinasen (50 mg bis 600 mg) wie der PDGF-Rezeptor-Kinase (vgl. EP-A-564409, WO 98/35958, US 5093330, WO 2004/005281, und WO 2006/041976); oxLDL Antikörper und oxLDL Impfstoffe; apoA-1 Milano; ASA; und VCAM-1 Inhibitoren.

Beispiele für Kombinationspartner zur Behandlung der Herzinsuffizienz sind Beta-Blocker wie Atenolol, Bisoprolol, Celiprolol, und Metoprolol; Diuretika wie Hydrochlorothiazide, Chlortalidon, Xipamid, Furosemid, Piretanid, Torasemid, Spironolacton, Eplerenon, Amilorid und Triamteren; ACE Hemmer wie Ramipril, Lisinopril, Cilazapril, Quinapril, Captopril, Enalapril, Benazepril, Perindopril, Fosinopril und Trandolapril; sowie Angiotensin II Rezeptor Blocker (ARBs) wie Telmisartan, Candesartan, Valsartan, Losartan, Irbesartan, Olmesartan und Eprosartan; Herzglykoside wie Digoxin und Digitoxin; kombinierte Alpha/Beta-Blocker wie carvedilol; B-type natriuretic peptide (BNP) und von BNP abgeleitete Peptide und BNP-Fusionsprodukte. Besonders bevorzugte Beispiele sind Metoprolol in der Dosierung von 50 mg bis 200 mg täglich, Ramipril in der Dosierung von 2,5 mg bis 15 mg täglich, Valsartan in der Dosierung von 80 bis 160 mg täglich, Telmisartan in der Dosierung 20 mg bis 320 mg oder 40 mg bis 160 mg täglich, Eplereron in der Dosierung 25-100 mg, Digoxin in der Dosierung 0,25 mg bis 0,6 mg täglich Carvedilol in der Dosierung 3,25 mg bis 100 mg, BNP (z.B. Nesiritide) in der Dosierung 2 µg/kg als Bolus gefolgt von 0,01 µg/kg/min.

Arzneimittelkombinationen der ausgewählten DPP IV Inhibitoren enthalten beispielsweise 1,75 mg bis 10,5 mg Glibenclamide, 500 mg bis 3000 mg Tolbutamide, 0,5-6 g Glimepiride, 2,5 mg bis 40 mg Glipizid, 1 -4x 30 mg Gliquidon, bis 3 x 25 mg Glibornurid, 80 mg bis 160 mg Gliclazid; 500 mg bis 1000 mg, bevorzugt 500 mg, 850 mg oder 1000 mg Metformin; 60 mg bis180 mg Nateglinide; 0,25 mg bis 4 mg Repaglinide; 2 mg bis 45 mg Thiazolidinedione; 200 mg bis 600 mg Metaglidasen; 2,5 mg bis 5 mg PPAR gamma/alpha Modulatoren; 0,1 mg bis 100 mg Alpha Glucosidase Blocker;1-250 IU Insulin; 15 µg bis120 µg Pramlintide; 5 mg bis 80 mg Statin; 50 mg bis 1000 mg Fibrat; 1000 mg bis 3000 mg Nikotinsäure oder - Derivat; etwa 250 mg Acipimox; etwa 10 mg eines Cholesterin Resorptionshemmers; 0,5 g bis 30 g einer Gallensäure bindenen Substanz; 10 mg bis 600 mg und bevorzugt 10 mg bis 120 mg CETP Inhibitor; 2,5 mg bis 100 mg Beta-Blocker; 3 mg bis 200 mg Diuretikum; 2,5 mg bis 500 mg Calcium Kanal Blocker; 1 mg bis 40 mg ACE Hemmer; 5 mg bis 600 mg Angiotensin II Rezeptor Blocker; 10 mg bis 15 mg Sibutramine; etwa 120 mg Orlistat; 15 mg bis 30 mg Dexfenfluramine; oder 5 mg bis 20 mg Cannabinoid Rezeptor Antagonist, Eplerenon in der Dosierung 25 mg bis 100 mg; Digoxin in der Dosierung 0,25 mg bis 0,6 mg täglich; Carvedilol in der Dosierung 3,25 mg bis 100 mg; BNP (z.B. Nesiritide) in der Dosierung 2 µg/kg als Bolus gefolgt von 0,01 µg/kg/min.

### BEISPIELE

### Beispiel 1: Behandlung eines Prä-Diabetes

Die Wirksamkeit eines erfindungsgemäßen DPPIV Inhibitors in der Therapie eines Prä-Diabetes gekennzeichnet durch pathologische Nüchternglukose und/oder gestörte Glukose-Toleranz lässt sich mittels klinischer Studien überprüfen. In Studien kürzerer Laufzeit (z.B. 2-4 Wochen) wird der Behandlungserfolg überprüft, indem die Nüchternglukosewerte und/oder die Glukosewerte nach einer Mahlzeit oder nach einem Belastungstest (oraler Glukosetoleranztest oder Nahrungstoleranztest nach einer definierten Mahlzeit) nach Ende der Studientherapiedauer bestimmt und mit den Werten vor Beginn der Studie und/oder mit denen einer Placebogruppe verglichen wird. Außerdem kann der Fruktosaminwert vor und nach Therapie bestimmt und mit dem Ausgangswert und/oder Placebowert verglichen werden. Ein signifikanter Abfall der Nüchtern- oder Nichtnüchtern-Glukosespiegel belegt die Wirksamkeit der Therapie. Bei Studien längerer Dauer (12 Wochen und mehr) wird der Therapieerfolg mittels Bestimmung des HbA1c-Wertes überprüft und zwar durch Vergleich mit dem Ausgangswert und/oder mit dem Wert der Placebogruppe. Eine signifikante Veränderung des HbA1c-Wertes im Vergleich zum Ausgangswert und/oder Placebowert belegt die Wirksamkeit des DPP IV Inhibitors in der Therapie des Prä-Diabetes.

### Beispiel 2: Vermeidung eines manifesten Typ 2 Diabetes

Eine Behandlung von Patienten mit pathologischer Nüchternglukose und/oder gestörter Glukose-Toleranz (Prä-Diabetes) verfolgt auch das Ziel, den Übergang zu einem manifesten Typ 2 Diabetes zu verhindern. Die Wirksamkeit einer Therapie kann in einer klinischen Vergleichsstudie untersucht werden, bei der Prä-Diabetes Patienten über einen längeren Zeitraum (z.B. 1 -5 Jahre) entweder mit einem Wirkstoff bzw. einer Wirkstoffkombination oder mit Placebo oder mit einer nicht-medikamentösen Therapie oder mit anderen Medikamenten behandelt. Während und am Ende der Therapie wird mittels Bestimmung der Nüchternglukose und/oder einem Belastungstest (z.B. oGTT) überprüft, wie viele Patienten einen manifesten Typ 2 Diabetes, d.h. einen Nüchternglukosespiegel >125 mg/dl und/oder 2h-Wert nach oGTT von >199 mg/dl, zeigen. Eine signifikante Reduktion der Zahl der Patienten, die bei der Behandlung mit Wirkstoff bzw. einer Wirkstoffkombination im Vergleich zu einer der anderen Therapieformen einen manifesten Typ 2 Diabetes zeigen, belegt die Wirksamkeit des Wirkstoffes bzw. der Wirkstoffkombination in der Verhinderung eines Überganges von Prä-Diabetes zu manifestem Diabetes.

### Beispiel 3: Behandlung eines Typ 2 Diabetes

Eine Behandlung von Patienten mit Typ 2 Diabetes mit den erfindungsgemäßen Wirkstoffen verhindert neben der akuten Verbesserung der Glukose-Stoffwechsellage langfristig auch eine Verschlechterung der Stoffwechsellage. Dies kann beobachtet werden, wenn Patienten über einen längeren Zeitraum, z.B. 1-6 Jahre, mit den erfindungsgemäßen Wirkstoffen bzw. Wirkstoffkombinationen behandelt und mit Patienten verglichen werden, die mit anderen antidiabetischen Medikamenten behandelt werden. Ein Nachweis des Therapieerfolges im Vergleich zu Patienten, die mit anderen antidiabetischen Medikamenten behandelt werden, liegt dann vor, wenn kein oder nur ein geringerer Anstieg der Nüchternglukose- und/oder HbA1 c-Werte beobachtet wird. Ein anderer Nachweis des Therapieerfolges liegt dann vor, wenn bei einem signifikant geringeren Prozentsatz der mit einem erfindungsgemäßen Wirkstoff bzw. einer erfindungsgemässen Wirkstoffkombination behandelten Patienten im Vergleich zu den Patienten, die mit anderen Medikamenten behandelt werden, einederartige Verschlechterung der Glukose-Stoffwechsellage eintritt (z.B. eine Erhöhung des HbA1c-Wertes auf >6.5% oder >7%), dass eine Therapie mit einem zusätzlichen oralen antidiabetischen Medikament oder mit Insulin oder mit einem Insulin-Analogon oder mit einem anderen antidiabetischem Prinzip (z.B. GLP-1 Analogon) angezeigt ist.

### Beispiel 4: Behandlung einer Insulin -Resistenz

In klinischen Studien unterschiedlicher Laufzeit (z.B. 2 Wochen bis 12 Monate) wird der Behandlungserfolg mittels einer hyperinsulinämischen euglykämischen Glukose-Clamp-Studie überprüft. Ein signifikanter Anstieg der Glukoseinfusionsrate am Ende der Studie im Vergleich zum Ausgangswert bzw. im Vergleich zu einer Placebogruppe, oder zu einer Gruppe, die einer anderen Therapie zugeführt wird, belegt die Wirksamkeit eines Wirkstoffes bzw. einer Wirkstoffkombination in der Therapie einer Insulin-Resistenz.

### Beispiel 5: Behandlung einer diabetischen Hyper- oder Dyslipidämie

In klinischen Studien unterschiedlicher Laufzeit (z.B. 2 Wochen bis 60 Monate) an Patienten mit Typ 2 Diabetes wird der Behandlungserfolg mittels Bestimmung von Gesamt-Cholesterin, LDL-Cholesterin, HDL-Cholesterin, und Plasma-Triglyzeriden überprüft. Ein signifikanter Abfall von Gesamt-Cholesterin, LDL-Cholesterin, oder Plasma-Triglyzeriden und/oder ein Anstieg der HDL-Cholesterinspiegel im Verlauf oder am Ende der Studie im Vergleich zum Ausgangswert bzw. im Vergleich zu einer Placebogruppe, oder zu einer Gruppe, die einer anderen Therapie zugeführt wird, belegt die Wirksamkeit eines Wirkstoffes bzw. einer Wirkstoffkombination in der Therapie einer diabetischen Dys- oder Hyperlipidämie.

### Beispiel 6: Behandlung einer Hyperglykämie

In klinischen Studien unterschiedlicher Laufzeit (z.B. 1 Tag bis 24 Monate) wird der Behandlungserfolg bei Patienten mit Hyperglykämie mittels Bestimmung der Nüchternglukose oder Nicht-Nüchternglukose (z.B. nach einer Mahlzeit oder eines Belastungs-Testes mit oGTT oder definierter Mahlzeit) überprüft. Ein signifikanter Abfall dieser Glukosewerte im Verlauf oder am Ende der Studie im Vergleich zum Ausgangswert bzw. im Vergleich zu einer Placebogruppe, oder zu einer Gruppe, die einer anderen Therapie zugeführt wurde, belegt die Wirksamkeit eines Wirkstoffes bzw. einer Wirkstoffkombination in der Therapie einer Hyperglykämie.

### Beispiel 7: Behandlung eines Gestations-Diabetes

In klinischen Studien kürzerer Laufzeit (z.B. 2-4 Wochen) wird der Behandlungserfolg überprüft, indem bei die Nüchternglukosewerte und/oder die Glukosewerte nach einer Mahlzeit oder nach einem Belastungstest (oraler Glukosetoleranztest oder Nahrungstoleranztest nach einer definierten Mahlzeit) am Ende der Studientherapiedauer bestimmt und mit den Werten vor Beginn der Studie u nd/oder mit denen einer Placebogruppe vergleicht. Außerdem kann der Fruktosaminwert vor und nach Therapie bestimmt und mit dem Ausgangswert und/oder Placebowert verglichen werden. Ein signifikanter Abfall der Nüchtern- oder Nichtnüchtern-Glukosespiegel belegt die Wirksamkeit eines Wirkstoffes bzw. einer Wirkstoffkombination.

Bei Studien längerer Dauer (12 Wochen und mehr) wird der Therapieerfolg mittels Bestimmung des HbA1c-Wertes (Vergleich mit Ausgangswert und mit Placebogruppe) überprüft. Eine signifikante Veränderung des HbA1c-Wertes im Vergleich zum Ausgangswert und/oder Placebowert belegt die Wirksamkeit eines Wirkstoffes bzw. einer Wirkstoffkombination in der Therapie eines Gestations-Diabetes.

### Beispiel 8: Behandlung von Frauen mit stattgehabtem Gestations-Diabetes

Patientinnen mit Gestations-Diabetes haben nach der Schwangerschaft ein deutlich erhöhtes Risiko, in der Folgezeit an einem manifesten Typ 2 Diabetes zu erkranken. Eine Therapie kann mit dem Ziel erfolgen, den Übergang zu einem manifesten Typ 2 zu verhindern. Zu diesem Ziel werden Frauen mit einem Gestations-Diabetes in der Vorgeschichte über einen längeren Zeitraum (z.B. 1-4 Jahre) entweder mit einem erfindungsgemäßen Wirkstoff bzw. einer erfindungsgemäßen Wirkstoffkombination oder mit Placebo oder mit einer nicht-medikamentösen Therapie oder mit anderen Medikamenten behandelt. Während und am Ende der Therapie wird mittels Bestimmung der Nüchternglukose und/oder einem Belastungstest (z.B. oGTT) überprüft, wie viele Patientinnen einen manifesten Typ 2 Diabetes (Nüchternglukosespiegel >125 mg/dl und/oder 2h-Wert nach oGTT >199 mg/dl) entwickelt haben. Eine signifikante Reduktion der Zahl der Patientinnen, die bei Behandlung mit einem erfindungsgemäßen Wirkstoff bzw. einer erfindungsgemäßen Wirkstoffkombination im Vergleich zu einer anderen Therapieform einen manifesten Typ 2 Diabetes entwickeln, ist ein Nachweis für die Wirksamkeit eines Wirkstoffs bzw. einer Wirkstoffkombination in der Verhinderung eines manifestem Diabetes bei Frauen mit einem Gestations-Diabetes in der Vorgeschichte.

### Beispiel 9: Prävention mikro- oder makrovaskulärer Komplikationen

Eine Behandlung von Typ 2 Diabetes oder Prä-Diabetes Patienten mit einem erfindungsgemäßen Wirkstoff bzw. einer erfindungsgemäßen Wirkstoffkombination verhindert oder verringert mikrovaskuläre Komplikationen (z.B. diabetische Neuropathie, diabetische Retinopathie, diabetische Nephropathie, diabetischer Fuß, diabetisches Ulcus) oder makrovaskuläre Komplikationen (z.B. Myokardinfarkt, akutes Koronar-Syndrom, instabile Angina pectoris, stabile Angina pectoris, Schlaganfall, periphere arterielle Verschlusskrankheit, Kardiomyopathie, Herzinsuffizienz, Herzrhythmusstörungen, vaskuläre Restenose). Typ 2 Diabetes oder mit Prä-Diabetes Patienten werden über einen längeren Zeitraum, z.B. 1-6 Jahre mit einem erfindungsgemäßen Wirkstoff bzw. einer erfindungsgemäßen Wirkstoffkombination behandelt und mit Patienten verglichen, die mit anderen antidiabetischen Medikamenten oder mit Placebo behandelt werden. Einen Nachweis des Therapieerfolges im Vergleich zu Patienten, die mit anderen antidiabetischen Medikamenten oder mit Placebo behandelt werden, erkennt man an der geringeren Anzahl einzelner oder mehrerer zusammengefasster Komplikationen. Bei makrovaskulären Ereignissen, diabetischem Fuß und/oder diabetischem Ulcus wird die Anzahl anhand der Anamnese und verschiedener Untersuchungsmethoden gezählt. Bei der diabetischen Retinopathie wird der Behandlungserfolg mittels Computer-gesteuerter Ablichtung und Auswertung des Augenhintergrundes oder anderen augenärztlichen Methoden festgestellt Bei der diabetischen Neuropathie kann neben der Anamnese und klinischen Untersuchung z.B. mittels geeichter Stimmgabel, eine Messung der Nervenleitgeschwindigkeit erfolgen. Bezüglich der diabetischen Nephropathie können folgende Parameter vor Beginn, im Verlauf und am Ende der Studie überprüft werden: Albuminausscheidung, Kreatinin-Clearance, Serum-Kreatininwerte, Zeit bis Verdopplung der Serum-Kreatinin-Werte, Zeit bis zur Dialysepflichtigkeit.

### Beispiel 10: Behandlung eines Metabolischen Syndroms

Die Wirksamkeit der erfindungsgemäßen Wirkstoffe bzw. Wirkstoffkombinationen lässt sich in klinischen Studien unterschiedlicher Laufzeit (z.B. 12 Wochen bis 6 Jahre) über die Bestimmung der Nüchternglukose oder Nicht-Nüchternglukose (z.B. nach einer Mahlzeit oder eines Belastungs-Testes mit oGTT oder definierter Mahlzeit) bzw. des HbA1c-Wertes überprüfen. Ein signifikanter Abfall dieser Glukosewerte bzw. HbA1c-Werte im Verlauf oder am Ende der Studie im Vergleich zum Ausgangswert bzw. im Vergleich zu einer Placebogruppe, oder zu einer Gruppe, die einer anderen Therapie zugeführt wurde, belegt die Wirksamkeit eines Wirkstoffs bzw. einer Wirkstoffkombinationen in der Therapie eines Metabolischen Syndroms. Einen positive n Einfluss auf das Metabolische Syndrom erkennt man auch an einer Verbesserung anderer Kennzeichen des Metabolischen Syndroms. Beispiele hierfür sind eine Absenkung des systolischen und/oder diastolischen Blutdruckes, eine Absenkung der Plasma-Triglyzeride, eine Absenkung des Gesamt- oder LDL-Cholesterins, eine Erhöhung des HDL-Cholesterins oder eine Reduktion des Gewichtes, entweder im Vergleich zum Ausgangswert zu Beginn der Studie oder im Vergleich zu einer Patientengruppe, die mit Placebo oder mit einer anderen Therapie behandelt werden.

### Beispiel 11: DPPIV Inhibitor Filmtabletten

Für die Herstellung einer Granulationslösung wird Copovidone bei Raumtemperatur in gereinigtem Wasser gelöst. DPP IV Inhibitor, Mannitol, vorgelierte Stärke und Maisstärke werden in einem geeigneten Mischer gemischt, um eine Vormischung herzustellen. Die Vormischung wird mit der Granulationslösung befeuchtet und anschließend in einem Mischer mit hoher Scherung granuliert. Das feuchte Granulat wird durch ein Sieb mit einer Mesh Größe von 1,6 mm gesiebt. Das Granulat wird bei etwa 60°C in einem Fliessbetttrockner getrocknet bis ein Verlust des Trocknungswertes von 2-4 % erreicht wird. Die endgültige Mischung wird zu Tablettenkernen verpresst.

In einem geeigneten Mischer werden bei Raumtemperatur Hydroxypropylmethylcellulose, Polyethylenglycol, Talk, Titandioxid und Eisenoxid in gereinigtem Wasser suspendiert, um einem Suspension für den Tablettenüberzug herzustellen. Die Tablettenkerne werden mit dieser Suspension beschichtet, bis eine Gewichtszunahme von 3% erreicht ist. Zum Beispiel folgende Tablettenzusammensetzungen können dabei erhalten werden:

| **Bestandteil** | **mg** | **mg** | **mg** | **mg** | **mg** |
|---|---|---|---|---|---|
| DPP IV Inhibitor | 0,500 | 1,000 | 2,500 | 5,000 | 10,000 |
| Mannitol | 67,450 | 66,950 | 65,450 | 130,900 | 125,900 |
| Vorgelierte Stärke | 9,000 | 9,000 | 9,000 | 18,000 | 18,000 |
| Maisstärke | 9,000 | 9,000 | 9,000 | 18,000 | 18,000 |
| Copovidone | 2,700 | 2,700 | 2,700 | 5,400 | 5,400 |
| Magnesiumstearat | 1,350 | 1,350 | 1,350 | 2,700 | 2,700 |
| **Gesamtmasse (Tablettenkern)** | **90,000** | **90,000** | **90,000** | **180,000** | **180,000** |
| HPMC | 1,500 | 1,500 | 1,500 | 2,500 | 2,500 |
| PEG | 0,150 | 0,150 | 0,150 | 0,250 | 0,250 |
| Titandioxid | 0,750 | 0,750 | 0,750 | 1,250 | 1,250 |
| Talk | 0,525 | 0,525 | 0,525 | 0,875 | 0,875 |
| Eisenoxid, gelb | 0,075 | 0,075 | 0,075 | 0,125 | 0,125 |
| **Gesamtmasse (Filmtablette)** | **93,000** | **93,000** | **93,000** | **185,000** | **185,000** |

### Beispiel 12: Stärkung der Vitalität und Sekretionskapazität von Langerhansschen Inseln oder Beta Zellen

Dies geschieht indem nach erfolgreicher Isolation der Langerhansschen Inseln oder pankreatischen Beta-Zellen diese bis zur vorgesehenen Transplantation in einem Medium, das DPP IV Inhibitoren in einer Konzentration von 1 nmol/l bis 1 µmol/l vorzugsweise in einer Konzentration von 1 nmol/l und 100 nmol/l enthält, aufbewahrt, transportiert oder kultiviert werden.

Des weiteren werden die Patienten (das können auch Tiere sein) nach erfolgter Transplantation von Langerhansschen Inseln oder pankreatischen Beta-Zellen mit DPP IV Inhibitoren in einer täglichen Dosis zwischen 1 mg und 200 mg, vorzugsweise mit einer Dosis von 5 mg und 100 mg eines DPP IV Inhibitors behandelt, um die Vitalität und Sekretionskapazität des Transplantats zu stärken. Geprüft wird das entweder durch eine Analyse der Insulinsekretion nach einer Stimulation mit Glucose oder einem anderen, die Insulinsekretion steigernden Agens. Zudem kann in vitro oder auch in Tiermodellen die Verbesserung der Qualität mit der TUNEL Technik, die in Diabetologia 42:566, 1999 oder Diabetes 48:738, 1999 beschrieben ist, nachgeprüft werden (Untersuchung der Apoptose und deren Inhibition).

### Beispiel 13: Kombinationsbehandlung DPP IV Inhibitor - Metformin

In der Behandlung eines Typ 2 Diabetes oder eines Prä-Diabetes kann ein erfindungsgemäßer DPP IV Inhibitor mit der anti-diabetisch wirksamen Substanz Metformin kombiniert werden, entweder in freier Kombination oder in einer fixierten Kombination in einer Tablette. Eine therapeutisch wirksame Dosis des DPP IV Inhibitors (z.B. eine Dosis zwischen 0,1 und 100 mg) kann mit verschiedenen Dosierungen von Metformin kombiniert werden, z.B. mit 500 mg, 850 mg oder 1000 mg Metformin als Einzeldosis bei einer Metformin Tages-Gesamtdosis von 500 -2850 mg, bzw. mit 500 mg, 1000 mg, 1500 mg, oder 2000 mg Metformin in retardierter Form. Die klinische Wirksamkeit einer solchen Kombination mit Metformin überprüft man in einer klinischen Studie. Dazu werden Patienten mit Typ 2 Diabetes oder mit Prä-Diabetes entweder mit einem DPP IV Inhibitor alleine oder mit Metformin alleine oder mit einer Kombination von DPP IV Inhibitor und Metformin behandelt. Die The rapiedauer beträgt zwischen 2 Wochen und 6 Jahren. Den Nachweis, dass die Kombination sinnvoll und wirksam ist, erkennt man darin, dass die Kombination eines DPP-IV Inhibitors mit Metformin zu einer signifikant stärkeren Absenkung der Nüchternglukose und/oder der Nicht-Nüchternglukose und/oder des HbA1c-Wertes führt als entweder der DPP IV Inhibitor alleine oder Metformin alleine.

### Beispiel 14: Kombinationsbehandlung DPP IV Inhibitor - Glitazone

In der Behandlung eines Typ 2 Diabetes oder eines Prä-Diabetes kann ein erfindungsgemäßer DPP IV Inhibitor mit der anti-diabetisch wirksamen Substanzgruppe der Glitazone bzw. Thiazolidinedione (z.B. Pioglitazone oder Rosiglitazone) kombiniert werden, entweder in freier Kombination oder in einer fixierten Kombination in einer Tablette. Eine therapeutisch wirksame Dosis des DPP IV Inhibitors (z.B. eine Dosis zwischen 0,1 mg und 100 mg) kann mit verschiedenen Dosierungen von Pioglitazone (15 mg, 30 mg, oder 45 mg) oder Rosiglitazone (2 mg, 4 mg oder 8 mg, entweder einmal oder zweimal täglich gegeben) kombiniert werden. Die klinische Wirksamkeit einer solchen Kombination mit Rosiglitazone oder Pioglitazone überprüft man in einer klinische n Studie. Dazu werden Patienten mit Typ 2 Diabetes oder mit Prä-Diabetes entweder mit einem DPP IV Inhibitor alleine oder mit Rosiglitazon bzw. Pioglitazon alleine oder mit einer Kombination von DPP IV Inhibitor und Rosiglitazon bzw. Pioglitazon behandelt. Die Therapiedauer beträgt zwischen 2 Wochen und 6 Jahren. Den Nachweis, dass die Kombination sinnvoll und wirksam ist, erkennt man darin, dass die Kombination des DPP-IV Inhibitors mit Rosiglitazon bzw. Pioglitazon zu einer signifikant stärkeren Absenkung der Nüchternglukose und/oder der Nicht-Nüchternglukose und/oder des HbA1c-Wertes führt als entweder der DPP IV Inhibitor alleine oder Rosiglitazon bzw. Pioglitazon alleine.

### Beispiel 15: Kombinationsbehandlung DPP IV Inhibitor - SGLT2 Inhibitor

In der Behandlung eines Typ 2 Diabetes oder eines Prä-Diabetes kann ein erfindungsgemäßer DPP IV Inhibitor mit der anti-diabetisch wirksamen Substanzgruppe der SGLT-2 Inhibitoren kombiniert werden, entweder in freier Kombination oder in einer fixierten Kombination in einer Tablette. Eine therapeutisch wirksame Dosis des DPP IV Inhibitors (z.B. eine Dosis zwischen 0,1 mg und 100 mg) kann mit verschiedenen Dosierungen eines SGLT-2 Inhibitors (0.5 mg bis 1000 mg) kombiniert werden. Die klinische Wirksamkeit einer solchen Kombination mit SGLT-2 Inhibitoren überprüft man in einer klinischen Studie. Dazu werden Patienten mit Typ 2 Diabetes oder mit Prä-Diabetes entweder mit einem DPP IV Inhibitor alleine oder mit einem SGLT-2 Inhibitor alleine oder mit einer Kombination von DPP IV Inhibitor und SGLT-2 Inhibitor behandelt. Die Therapiedauer beträgt zwischen 2 Wochen und 6 Jahren. Den Nachweis, dass die Kombination sinnvoll und wirksam ist, erkennt man darin, dass die Kombination des DPP-IV Inhibitors mit dem SGLT-2 Inhibitor zu einer signifikant stärkeren Absenkung der Nüchternglukose und/oder der Nicht-Nüchternglukose und/oder des HbA1c-Wertes führt als entweder der DPP IV Inhibitor alleine oder der SGLT-2 Inhibitor alleine.

### Beispiel 16: Kombinationsbehandlung DPP IV Inhibitor - Antihypertensivum

In der Behandlung eines Patienten mit Typ 2 Diabetes oder mit Prä-Diabetes oder mit Metabolischem Syndrom kann ein erfindungsgemäßer DPP IV Inhibitor mit einer anti-hypertensiv wirksamen Substanz kombiniert werden, entweder in freier Kombination oder in einer fixierten Kombination in einer Tablette. Eine therapeutisch wirksame Dosis des DPP IV Inhibitors (z.B. eine Dosis zwischen 0,1 mg und 100 mg) kann mit verschiedenen Dosierungen von ACE-Hemmern (z.B. 2,5 mg bis15 mg Ramipril), AT1 - Rezeptor-Antagonisten (z.B. 20 mg bis160 mg Telmisartan), Beta-Blockern (z.B. 50 mg bis 200 mg Metoprolol), oder Diuretika (z.B. 12,5 mg bis 25 mg Hydrochlorothiazid) kombiniert werden. Die klinische Wirksamkeit einer solchen Kombination mit Antihypertensiva überprüft man in einer klinischen Studie. Dazu werden Patienten mit Typ 2 Diabetes oder mit Prä-Diabetes oder mit Metabolischem Syndrom entweder mit einem DPP IV Inhibitor alleine oder mit einem Antihypertensivum alleine oder mit einer Kombination von DPP IV Inhibitor und Antihypertensivum behandelt. Die Therapiedauer beträgt zwischen 2 Wochen und 6 Jahren. Den Nachweis, dass die Kombination sinnvoll und wirksam ist, erkennt man darin, dass die Kombination des DPP-IV Inhibitors mit dem Antihypertensivum den Nüchternglukose und/oder Nicht-Nüchternglukose und/oder HbA1 c-Wert mindeste ns so stark absenkt wie der DPP-IV Inhibitor alleine, und wenn die Kombination des DPP-IV Inhibitors mit dem Antihypertensivum den systolischen und/oder diastolischen arteriellen Blutdruck mindestens so stark absenkt wie das Antihypertensivum alleine.

### Beispiel 17: Kombinationsbehandlung DPP IV Inhibitor - Lipidsenker

In der Behandlung eines Patienten mit Typ 2 Diabetes oder mit Prä-Diabetes oder mit Metabolischem Syndrom oder mit diabetischer Dys- oder Hyperlipidämie kann ein erfindungsgemäßer DPP IV Inhibitor mit einem Lipidsenker / HDL-Erhöher kombiniert werden, entweder in freier Kombination oder in einer fixierten Kombination in einer Tablette. Eine therapeutisch wirksame Dosis des DPP IV Inhibitors (z.B. eine Dosis zwischen 0,1 mg und 100 mg) kann mit verschiedenen Dosierungen von Statinen (z.B. 10 mg bis 80 mg Atorvastatin oder 10 mg bis 80 mg Simvastatin), von Fibraten (z.B. Fenofibrat), von Cholesterinabsorptionshemmern, oder mit HDL-erhöhenden Substanzen wie CETP-Inhibitoren (z.B. Torcetrapib 10 mg bis120 mg einmal täglich oder 120 mg zweimal täglich) kombiniert werden. Die klinische Wirksamkeit einer solchen Kombination mit Lipidsenkern / HDL-Erhöhern überprüft man in einer klinischen Studie. Dazu werden Patienten mit Typ 2 Diabetes oder mit Prä-Diabetes oder mit Metabolischem Syndrom oder mit diabetischer Dys- oder Hyperlipidämie entweder mit einem DPP IV Inhibitor alleine oder mit einem Lipidsenker / HDL-Erhöher alleine oder mit einer Kombination von DPP IV Inhibitor und Lipidsenker / HDL-Erhöher behandelt. Die Therapiedauer beträgt zwischen 2 Wochen und 6 Jahren. Den Nachweis, dass die Kombination sinnvoll und wirksam ist, erkennt man darin, dass die Kombination des DPP-IV Inhibitors mit dem Lipidsenker / HDL-Erhöher den Nüchternglukose und/oder Nicht-Nüchternglukose und/oder HbA1 c-Wert mindestens so stark absenkt wie der DPP-IV Inhibitor alleine, und wenn die Kombination des DPP-IV Inhibitors mit einem Lipidsenker / HDL-Erhöher den Wert von Gesamtcholesterin oder LDL-Cholesterin oder Plasmatriglyzeriden mindestens so stark absenkt bzw. den HDL-Cholesterin Wert mindestens genauso stark erhöht wie der Lipidsenker / HDL-Erhöher alleine.

### Beispiel 18: Kombinationsbehandlung DPP IV Inhibitor - BNP / von BNP abgeleitete Peptide oder BNP-Fusionspeptide bei Patienten mit Herzinsuffizienz

In der Behandlung eines Patienten mit akuter Herzinsuffizienz kann ein erfindungsgemäßer DPP IV Inhibitor mit einer Substanz kombiniert werden, welche eine Herzinsuffizienz günstig beeinflusst, entweder in freier Kombination oder in einer fixierten Kombination in einer Tablette. Eine therapeutisch wirksame Dosis des DPP IV Inhibitors (z.B. eine Dosis zwischen 0,1 mg und 100 mg) kann mit verschiedenen Dosierungen von ACE-Hemmern (z.B. 2,5 mg bis15 mg Ramipril), AT1- Rezeptor-Antagonisten (z.B. 20 mg bis160 mg Telmisartan), Beta-Blockern (z.B. 50 mg bis 200 mg Metoprolol), kombinierter Alpha/Beta-Blocker (z.B. 3,25 mg bis 100 mg Carvedilol), Diuretika (z.B. 12,5 mg bis 25 mg Hydrochlorothiazid), Mineralokortikoidrezeptor-Antagonisten (z.B. 25 mg bis 100 mg Eplerenon; und/oder B-Typ Natriuretischem Peptid (BNP) (z.B. 2 µg/kg als Bolus gefolgt von 0,01 µg/kg/min Nesiritide), einem von BNP abgeleiteten Peptide oder einem BNP-Fusionsprodukte kombiniert werden. Die Kombination aus BNP und DPP-IV Inhibitor führt zu einer höheren Konzentration von BNP voller Länge (1-32) in vivo. Die klinische Wirksamkeit der angegebenen Kombinationen überprüft man in klinischen Studien. Die Therapiedauer beträgt zwischen 1 Tag und 6 Jahren. Den Nachweis, dass die Kombination in der Behandlung einer akuten Herzinsuffizienz wirksam ist, erkennt man darin, dass die Kombination im Vergleich zu einer anderen Therapie zu einer signifikanten Besserung der klinischen Situation führt (höhere Herzauswurfleistung und/oder Rückgang einer pulmonalen Stauung, und/oder Rückgang des pulmonalen Wedge-Druckes, und/oder Reduktion der akuten Herzinsuffizienz bedingten Mortalität).

### Beispiel 19: Behandlung mit DPP-IV inhibitor bei Patienten mit Herzinsuffizienz

In der Behandlung eines Patienten mit chronischer Herzinsuffizienz kann ein erfindungsgemäßer DPP IV Inhibitor eingesetzt werden. Diese Behandlung führt zu einer höheren Konzentration von endogenem BNP voller Länge (1-32) in vivo. Die klinische Wirksamkeit dieser Therapie überprüft man in klinischen Studien. Die Therapiedauer beträgt zwischen 2 Wochen und 6 Jahren. Den Nachweis, dass die Therapie in der Behandlung einer chronischen Herzinsuffizienz wirksam ist, erkennt man darin, dass ein erfindungsgemäßer DPP-IV Inhibitor im Vergleich zu einer anderen Therapie oder zu Placebo zu einer signifikanten Besserung der klinischen Situation führt (seltenere Hospitalisierung aufgrund einer akuten Herzinsuffizienz, längere Gehstrecke, höhere Belastbarkeit in der Ergometrie, höhere Herzauswurfleistung und/oder Rückgang einer pulmonalen Stauung, und/oder Reduktion der Herzinsuffizienz bedingten Mortalität).

## Patentansprüche

1. Verwendung eines DPP IV Inhibitors der Formel (I) 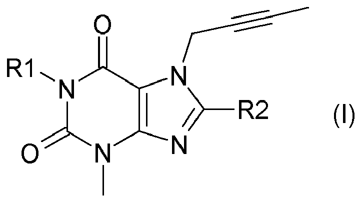 oder der Formel (II) 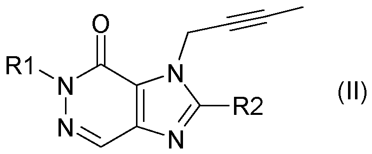 sowie eines seiner Salze, **dadurch gekennzeichnet, dass**
**R1** ([1,5]Naphthyridin-2-yl)methyl, (Chinazolin-2-yl)methyl, (Chinoxalin-6-yl)methyl, (4-Methyl-chinazolin-2-yl)methyl, 2-Cyano-benzyl, (3-Cyano-chinolin-2-yl)methyl, (3-Cyano-pyridin-2-yl)methyl, (4-Methyl-pyrimidin-2-yl)methyl, oder (4,6-Dimethyl-pyrimidin-2-yl)methyl bedeutet und
**R2** 3-(*R*)-amino-piperidin-1-yl, (2-amino-2-methyl-propyl)-methylamino oder (2-(S)-amino-propyl)-methylamino,
zur Herstellung einer Arzneimittelkombination mit Telmisartan.

2. Verwendung gemäß Anspruch 1, **dadurch gekennzeichnet, dass** die Arzneimittelkombination der therapeutischen Behandlung eines Patienten dient, bei dem eine physiologische Funktionsstörung diagnostiziert wurde, die ausgewählt ist aus der Gruppe Prä-Diabetes, Glukose-Intoleranz, pathologischer Nüchtern-Glukose, diabetischer Fuß, Diabetes-assoziierter Ulkus, diabetische Hyperlipidämie, diabetische Dyslipidämie, neu diagnostizierter Typ 1 Diabetes, Gestations-Diabetes, Herzinsuffizienz, Hyperglykämie und Adrenerges postprandiales Syndrom.

3. Ein DPP IV Inhibitor wie in Anspruch 1 definiert zur Verwendung in der Kombinationsbehandlung mit Telmisartan.

4. Ein DPP IV Inhibitor wie in Anspruch 1 definiert zur Verwendung in der Behandlung eines Patienten mit Typ 2 Diabetes oder mit Prä-Diabetes oder mit Metabolischem Syndrom, wobei der DPP IV Inhibitor mit Telmisartan kombiniert wird.

5. Ein DPP IV Inhibitor wie in Anspruch 1 definiert zur Verwendung nach Anspruch 3 oder 4, wobei die Kombinationsbehandlung als freie Kombination der Wirkstoffe erfolgt.

6. Ein DPP IV Inhibitor wie in Anspruch 1 definiert zur Verwendung nach Anspruch 3 oder 4, wobei die Kombinationsbehandlung in Form einer fixierten Kombination erfolgt.

7. Ein DPP IV Inhibitor wie in Anspruch 1 definiert zur Verwendung nach einem der Ansprüche 3 bis 6, wobei Telmisartan in einer Dosierung von 20 mg bis 160 mg, 20 mg bis 320 mg oder 40 mg bis 160 mg täglich verwendet wird.

8. Ein DPP IV Inhibitor wie in Anspruch 1 definiert in Kombination mir Telmisartan zur Verwendung in einem Verfahren zur Behandlung eines Prä-Diabetes oder Typ 2 Diabetes Patienten, **dadurch gekennzeichnet, dass** durch die Behandlung eine Verringerung des Risikos einer verschlechterten Glukose-Stoffwechsellage trotz Therapie, eines erhöhten HbA1c-Wertes trotz Therapie, einer Verschlechterung der Nüchternglukose trotz Therapie, der Notwendigkeit einer Insulin-Therapie, eines manifesten Typ 2 Diabetes, eines diabetischen Fußes, eines Diabetes-assoziierten Ulkus, einer diabetischen Hyperlipidämie, einer diabetischen Dyslipidämie oder einer makrovaskulären Komplikation erzielt wird.

9. Ein DPP IV Inhibitor wie in Anspruch 1 definiert in Kombination mir Telmisartan zur Verwendung in einem Verfahren zur Behandlung eines Patienten, **dadurch gekennzeichnet, dass** der Patient an Herzinsuffizienz leidet.
